Europäisches Patentamt

European Patent Office

Office européen des brevets

(11) Veröffentlichungsnummer: **0 096 438**
**A2**

(12) **EUROPÄISCHE PATENTANMELDUNG**

(21) Anmeldenummer: 83200765.2

(22) Anmeldetag: 31.05.83

(51) Int. Cl.³: **A 61 B 6/00**

(30) Priorität: 04.06.82 DE 3221043

(43) Veröffentlichungstag der Anmeldung:
21.12.83 Patentblatt 83/51

(84) Benannte Vertragsstaaten:
BE DE FR GB

(71) Anmelder: **Philips Patentverwaltung GmbH**
**Billstrasse 80**
**D-2000 Hamburg 28(DE)**

(84) Benannte Vertragsstaaten:
**DE**

(71) Anmelder: **N.V. Philips' Gloeilampenfabrieken**
**Groenewoudseweg 1**
**NL-5621 BA Eindhoven(NL)**

(84) Benannte Vertragsstaaten:
**BE FR GB**

(72) Erfinder: **Koropp, Norbert**
**Charlottenburger Weg 1**
**D-2057 Reinbek(DE)**

(74) Vertreter: **Hartmann, Heinrich, Dipl.-Ing. et al,**
**Philips Patentverwaltung GmbH Billstrasse 80 Postfach**
**10 51 49**
**D-2000 Hamburg 28(DE)**

(54) Anordnung zum Gewichtsausgleich für ein Geräteteil an einem um eine horizontale Achse schwenkbaren Röntgenuntersuchungsgerät.

(57) Die Erfindung betrifft einen Gewichtsausgleich für ein Röntgenuntersuchungsgerät mit einem als Gasfeder wirkenden Druckluftzylinder, dessen Innendruck in Abhängigkeit vom Sinus des Schwenkwinkels steuerbar ist. Zu diesem Zweck ist ein Druckregelkreis vorgesehen, dessen Führungsgröße von einem Sinusgeber geliefert wird, der ein vom Sinus des Schwenkwinkels abhängiges Signal erzeugt.

FIG.2

EP 0 096 438 A2

"Anordnung zum Gewichtsausgleich für ein Geräteteil an einem um eine horizontale Achse schwenkbaren Röntgenuntersuchungsgerät"

Die Erfindung betrifft eine Anordnung zum Gewichtsausgleich für Geräteteile an einem um eine horizontale Achse schwenkbaren Untersuchungsgerät mit einem das Geräteteil tragenden Druckluftzylinder, in dem eine Kolbenstange verschiebbar angeordnet ist, der eine Öffnung zum Be- und Entlüften aufweist und dessen Innendruck von einem Sinusgeber steuerbar ist, der ein dem Sinus des Schwenkwinkels entsprechendes Signal erzeugt.

Eine solche Anordnung ist aus der DE-AS 28 06 956 bekannt. Bei der bekannten Anordnung, die zugleich auch zum Verschieben des Geräteteils dient, wird der Druckluftzylinder durch den Kolben in zwei Kammern unterteilt, in denen unterschiedliche Drücke herrschen. Jede Kammer ist mit einer Öffnung versehen, durch die Luft zu- oder abgeführt werden kann, so daß das Geräteteil in verschiedenen Richtungen verschoben werden kann. Die dafür erforderliche pneumatische Steuervorrichtung ist recht kompliziert und teuer. Wenn die Druckluftquelle, mit der der Druckluftzylinder verbunden ist, einmal ausfällt, kann das Geräteteil nicht verschoben werden.

Weiterhin ist aus der DE-AS 25 57 810 ein Stativ für ein röntgenologisches Bilderfassungsgerät bekannt, bei dem ein verschiebbares Geräteteil von einer Gasfeder getragen wird. Zwar ist der Gewichtsausgleich dabei wesentlich einfacher als bei der eingangs genannten Anordnung, jedoch kann das Geräteteil nur vertikal verschoben werden.

Aufgabe der vorliegenden Erfindung ist es, eine Anordnung der eingangs genannten Art, bei der das Geräteteil je nach der Stellung des Untersuchungsgerätes, mit dem es verbunden ist, in verschiedenen Richtungen verschiebbar ist, zu vereinfachen.

Diese Aufgabe wird erfindungsgemäß dadurch gelöst, daß der Kolben so angeordnet und ausgebildet ist, daß der Druck im gesamten Druckluftzylinder homogen verteilt ist und daß ein Druckregelkreis vorgesehen ist, dessen Führungsgröße der Sinusgeber liefert.

Der Druckluftzylinder wirkt zusammen mit dem durch den Sinusgeber geführten Regelkreis wie eine Gasfeder, deren Gasdruck entsprechend der jeweiligen Stellung des Röntgenuntersuchungsgerätes geändert wird, so daß die Kräfte, die durch den Gasdruck im Zylinder und durch das Geräteteil auf den Kolben ausgeübt werden, einander in jeder Stellung des Geräteteils entsprechen und das Gewicht des Geräteteils ausgeglichen ist.

Eine Weiterbildung der Erfindung sieht vor, daß ein Meßglied zur Messung der vom Benutzer auf das Geräteteil ausgeübten Kraft vorgesehen ist, dessen Ausgangssignal der Führungsgröße überlagert ist.

Durch diese Maßnahme ergibt sich ein Servoantrieb, dessen besonderer Vorteil darin liegt, daß der Gasdruck und damit die auf den Kolben und damit auch auf das Geräteteil einwirkende Kraft der vom Benutzer aufgebrachten Kraft entspricht.

Eine andere Weiterbildung der Erfindung sieht vor, daß der Regelkreis eine mit dem Druckzylinder gekoppelte Ventilanordnung enthält mit einem Ventil zum Belüften und einem Ventil zum Entlüften des Zylinders, daß die beiden Ventile durch je einen Motor betätigbar sind, und daß mit jedem Motor und dem zugehörigen Ventil eine Feder so gekoppelt ist, daß sie beim Öffnen des Ventils durch den Motor gespannt wird.

Wenn in geöffneter Stellung eines der Ventile der zugehörige Motor z.B. durch einen Spannungsausfall unwirksam wird, wird das betreffende Ventil durch die vorher gespannte Feder geschlossen, so daß der im Druckluftzylinder herrschende Druck im wesentlichen erhalten bleibt.

Die Erfindung wird nachstehend anhand der Zeichnung näher erläutert. Es zeigen

Fig. 1 ein Röntgenuntersuchungsgerät mit einem erfindungsgemäßen Druckluftzylinder,

Fig. 2 ein Blockschaltbild eines solchen Zylinders und

Fig. 3 eine Darstellung eines im Regelkreis enthaltenen Stellgliedes.

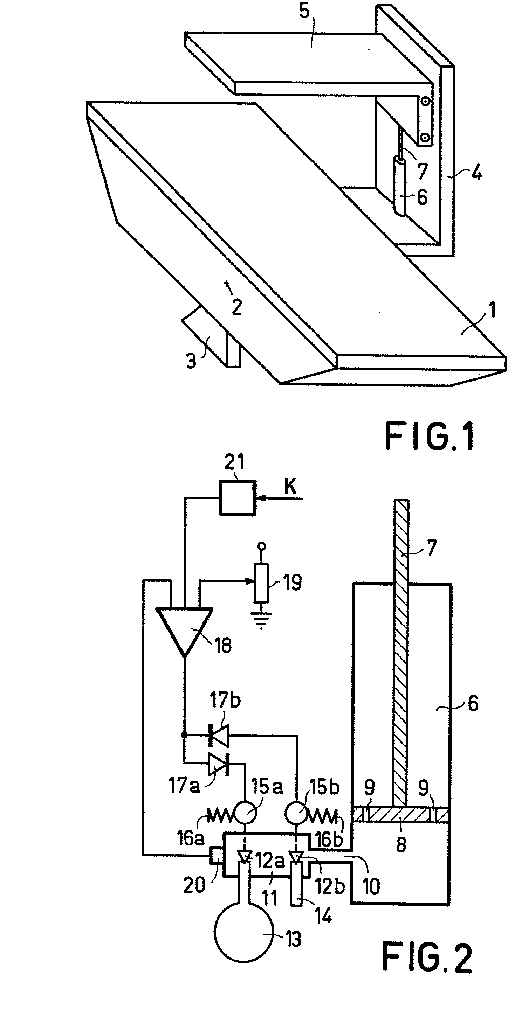

Das in Fig. 1 gezeichnete Röntgenuntersuchungsgerät enthält einen Rahmen 1 mit Patientenlagerungsplatte, der um eine horizontale Achse 2 am Fuß 3 des Gerätes schwenkbar ist. Innerhalb des Rahmens ist ein Längswagen 4 in einer zur Ebene der Patientenlagerungsplatte parallelen Ebene verschiebbar, und in nicht näher dargestellten Führungsschienen an dem Rahmen ist ein das Zielgerät tragender Wagen 5 senkrecht zur Tischplatte verschiebbar. An dem Längswagen 4 ist ein Druckluftzylinder 6 befestigt, in dem ein Kolben 7 verschiebbar angeordnet ist, der an seinem einen Ende mit dem Zielgerätewagen 5 verbunden ist. Der Druck in dem Kolben wird jeweils so geändert, daß das Zielgerätegewicht ausgeglichen ist. In einer horizontalen Stellung des Röntgenuntersuchungsgerätes muß der Druck in dem Kolben ein Maximum erreichen, so daß die Kraft auf den Kolben dem Zielgerätegewicht entspricht. In einer vertikalen Stellung des

Röntgenuntersuchungsgerätes hingegen darf keine Kraft auf den Kolben ausgeübt werden.

Wie sich aus Fig. 2 ergibt, ist an dem in Kolben hineinragenden Teil ein Führungsflansch 8 befestigt, der mit Bohrungen 9 versehen ist, durch die hindurch Gas bzw. Luft zwischen den beiden durch den Führungsflansch 8 voneinander getrennten Teilen des Druckzylinders fließen kann. Der Luftdruck ist daher überall innerhalb des Druckluftzylinders 6 gleich. Die von ihm auf den Kolben 7 ausgeübte Kraft entspricht der Querschnittsfläche des Kolbens multipliziert mit dem jeweils in dem Zylinder herrschenden Druck.

Der Druckluftzylinder steht über eine Öffnung 10 mit einer Ventilanordnung 11 in Verbindung, die zwei Ventile 12a bzw. 12b enthält. Wird das Ventil 12a geöffnet, strömt Luft aus einer Preßluftquelle 13, in der Luft unter einem Druck gespeichert ist, der wesentlich größer ist als der größte im Druckluftzylinder 6 erforderliche Druck, in den Zylinder 6 ein. Dabei erhöht sich der Druck im Zylinder. Wird hingegen das Ventil 12b geöffnet, so fließt Luft aus dem Druckluftzylinder 6 über eine Leitung 14 ins Freie ab. Zur Vermeidung von Geräuschen kann dabei an der Leitung 14 ein Schalldämpfer angebracht sein.

Die Stellung der Ventile 12a bzw. 12b wird durch je einen Motor 15a bzw. 15b bestimmt, der das zugeordnete Ventil gegen die Kraft einer Feder 16a bzw. 16b öffnen kann. Die Motoren 15a und 15b sind mit ihrem einen Anschluß über Dioden 17a und 17b mit einander entgegengesetzter Durchlaßrichtung mit dem Ausgang eines Verstärkers 18 verbunden und mit ihrem anderen Anschluß an ein Potential angeschlossen, das dem Ruhepotential, d.h. dem Potential am Ausgang bei fehlendem Eingangssignal des Verstärkers 18, entspricht. An den Eingang des Verstärkers 18 ist der Abgriff eines Sinuspotentiometers 19 angeschlossen, das so mit dem Rahmen 1 des Untersuchungsgerätes verbunden ist, das sich die Spannung an dem Abgriff sinusförmig mit dem Schwenkwinkel des Untersuchungsgerätes ändert. Die Spannung an diesem Abgriff entspricht daher bei jeder Schwenkstellung des Gerätes der vom Zielgerätewagen 5 auf den Kolben 7 ausgeübten Kraft, die - bei horizontaler Stellung des Untersuchungsgerätes - bis zu 2.000 N betragen kann. Mit einem anderen Eingang des Verstärkers 18 ist ein Drucksensor 20 verbunden, der ein dem Druck in dem Druckluftzylinder 6 proportionales Signal liefert. Der Verstärker 18 bildet ein der Differenz zwischen diesen beiden Signalen entsprechendes Signal, und zwar derart, daß das Potential am Ausgang des Verstärkers 18 negativ wird, wenn das vom Drucksensor 20 gelieferte Signal größer ist als das Signal am Abgriff des Sinuspotentiometers 19 und daß das Potential am Ausgang des Verstärkers 18 positiver wird, wenn das vom Drucksensor 20 gelieferte Signal kleiner ist als das am Abgriff des Sinuspotentiometers abgegriffene Signal.

Infolgedessen wird die Diode 17b leitend, wenn der Druck in dem Zylinder 6 zu groß wird, wodurch der Motor 15b aktiviert wird, der das Ventil 12b öffnet, so daß Luft abfließt und der Druck abnimmt. Dabei spannt der Motor 15b gleichzeitig die Feder 16b vor. Wenn der Druck im Zylinder 6 so weit abgenommen hat, daß das vom Drucksensor 20 gelieferte Signal der Spannung am Abgriff des Sinuspotentiometers 19 entspricht, wird die Diode 17b gesperrt. Das Ventil 12b wird dann durch die vom Motor 15b vorgespannte Feder 16b geschlossen und der Druck bleibt konstant. Ist der Druck in dem Druckzylinder niedriger als der am Potentiometerabgriff abnehmbaren Spannung entspricht - was beispielsweise dann passieren kann, wenn das Röntgenuntersuchungsgerät in eine horizontale Position geschwenkt wird - wird das Potential am Ausgang des Verstärkers 18 positiver, so daß die Diode 17a leitend wird und der Motor 15a läuft, wobei das Ventil 12a geöffnet wird und die Feder 16a gespannt wird. Dabei strömt Luft aus der Druckluftquelle 13 in den Druckluftzylinder 6 ein, bis der Druck darin so groß geworden ist, daß die Spannungen an den Ausgängen der beiden Wandler 19 und 20 einander gleich sind. Danach wird der Motor 15a wieder stromlos, wonach das Ventil 12a durch die Feder 16a wieder geschlossen wird.

Auf diese Weise ergibt sich ein Regelkreis, dessen Führungsgröße durch die Spannung am Abgriff des Sinuspotentiometers 19 gegeben wird. Durch geeignete Abschwächung oder Verstärkung der vom Drucksensor 20 und/oder vom Sinuspotentiometer 19 gelieferten Signale läßt sich dabei erreichen, daß in jeder Schwenkstellung des Röntgenuntersuchungsgerätes die von der komprimierten Luft im Innern des Druckluftzylinders 6 auf den Kolben 7 ausgeübte Kraft genauso groß ist wie die vom Geräteteil auf den Kolben ausgeübte Kraft. Dadurch ergibt sich ein Gewichtsausgleich ohne Gegengewicht.

Grundsätzlich kann der Benutzer das Geräteteil von Hand bewegen, weil nur noch die Beschleunigungs- und Reibungskräfte für die Bewegung des Geräteteils zu überwinden sind. Mit geringem zusätzlichen Aufwand ist jedoch auch eine servomotorische Verschiebung des Zielgerätes möglich, wobei die vom Benutzer aufgebrachte Kraft kleiner sein kann als zur Beschleunigung des Geräteteils und zur Überwindung der Reibung erforderlich. Zu diesem Zweck ist ein Wandler 21 vorgesehen, der die vom Benutzer - z.B. an einem Handgriff - auf das Geräteteil ausgeübte Kraft in ein elektrisches Signal umwandelt, das einem Eingang des Verstärkers zugeführt wird und der vom Sinuspotentiometer 19 gelieferten Führungsgröße so überlagert wird, daß der Druck im Druckluftzylinder 6 je nach Richtung der Kraft auf das Geräteteil entweder zunimmt oder abnimmt. Wenn das

0096438
PHD 82-066 EP

Verhältnis von der vom Wandler 21 gemessenen, vom Benutzer ausgeübten Kraft K zu dem vom Wandler 21 erzeugten elektrischen Signal konstant und unabhängig von der Kraft ist, ergibt sich eine Druckänderung in dem Druckluftzylinder 6, die der Kraft proportional ist, so daß der Kolben 7 mit einer der vom Benutzer ausgeübten Kraft proportionalen (aber größeren) Kraft nach außen oder nach innen gedrückt wird, wodurch die körperliche Arbeit des Benutzers erleichtert wird.

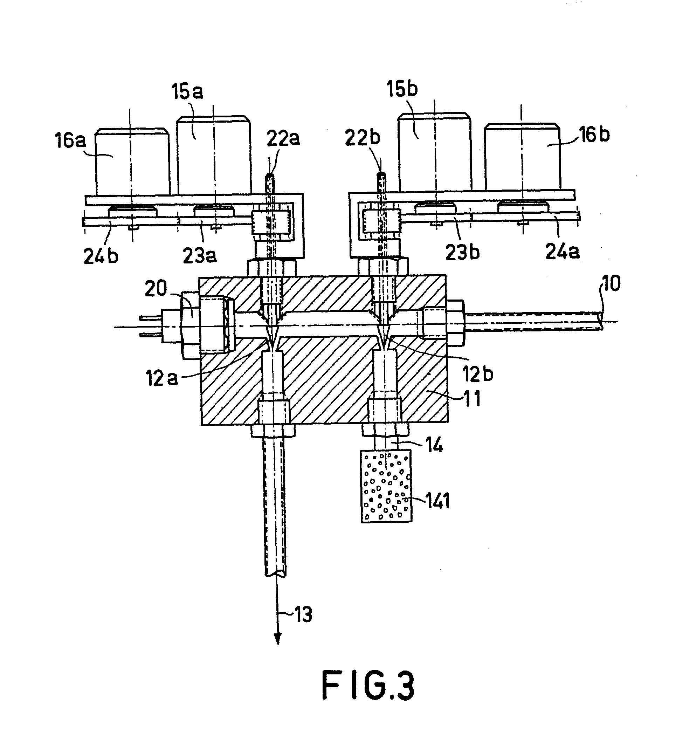

Wie aus Fig. 3 ersichtlich, besitzt die Ventilanordnung 11 zwei Ventile 12a und 12b, die die Form einer Kegelspitze haben und mit Hilfe einer Spindel 22a bzw. 22b in den ebenfalls kegelförmigen Ventilsitz hinein- oder aus ihm herausgedrückt werden können. Jede Spindel 22a bzw. 22b ist über ein Ritzel 23a bzw. 23b mit dem Motor 15a bzw. 15b gekoppelt, über das zugleich ein weiteres Ritzel 24a und 24b angetrieben wird. Mit den letztgenannten beiden Ritzeln sind die Federn 16a und 16b so gekoppelt, daß bei einem Öffnen des Ventils durch den Motor die bereits vorgespannte Feder weiter vorgespannt wird. Wenn der gewünschte Druck erreicht ist, so daß das Ausgangssignal des Verstärkers 18 Null ist, oder bei einem Stromausfall schließen die Federn 16a bzw. 16b sofort das zugehörige Ventil, falls dies noch geöffnet ist. Der Druck im Druckluftzylinder 6 bleibt also auch im Falle eines Stromausfalls erhalten, da er weitgehend unabhängig von der Stellung des Kolbens 7 ist (weil das von diesem verdrängte Luftvolumen klein im Vergleich zu dem Gesamtvolumen des Druckluftzylinders 6 ist, bleibt die auf den Kolben ausgeübte Kraft unabhängig von seiner Stellung im wesentlichen konstant) , so daß z.B. bei einem Stromausfall das Geräteteil in der erreichten Stellung des Untersuchungsgerätes ohne weiteres bewegt werden kann.

PATENTANSPRÜCHE:

1.   Anordnung zum Gewichtsausgleich für Geräteteile an einem um eine horizontale Achse schwenkbaren Untersuchungsgerät mit einem das Geräteteil tragenden Druckluftzylinder, in dem eine Kolbenstange verschiebbar angeordnet ist, der eine Öffnung zum Be- und Entlüften aufweist und dessen Innendruck von einem Sinusgeber steuerbar ist, der ein dem Sinus des Schwenkwinkels entsprechendes Signal erzeugt, dadurch gekennzeichnet, daß der Kolben (7) so angeordnet und ausgebildet ist, daß der Druck im gesamten Druckluftzylinder (6) homogen verteilt ist und daß ein Druckregelkreis (11...18, 20) vorgesehen ist, dessen Führungsgröße der Sinusgeber (19) liefert.

2.   Anordnung nach Anspruch 1, dadurch gekennzeichnet, daß ein Meßglied (21) zur Messung der vom Benutzer auf das Geräteteil (5) ausgeübten Kraft vorgesehen ist, dessen Ausgangssignal der Führungsgröße überlagert ist.

3.   Anordnung nach Anspruch 1, dadurch gekennzeichnet, daß der Regelkreis eine mit dem Druckzylinder gekoppelte Ventilanordnung enthält mit einem Ventil (12a) zum Belüften und einem Ventil zum Entlüften (12b) des Zylinders, daß die beiden Ventile (12a, 12b) durch je einen Motor (15a, 15b) betätigbar sind, und daß mit jedem Motor und dem zugehörigen Ventil eine Feder (16a, 16b) so gekoppelt ist, daß sie beim Öffnen des Ventils (12a, 12b) durch den Motor (15a, 15b) gespannt wird.

1/2

0096438

**FIG.1**

**FIG.2**

1-II-PHD 82-066

FIG.3